# EUROPEAN PATENT SPECIFICATION

(11) **EP 4 237 818 B1**
(45) Date of publication and mention of the grant of the patent: **09.12.2026**
(21) Application number: 21884225.0
(22) Date of filing: 28.10.2021
(51) Int. Cl.: G01N 1/28, C12N 15/10, C12Q 1/6806, C12N 1/04

(54) **FIXATIVE COMPOSITIONS AND METHODS OF PRESERVING BIOLOGICAL SAMPLES**
FIXIERUNGSZUSAMMENSETZUNGEN UND VERFAHREN ZUR KONSERVIERUNG BIOLOGISCHER PROBEN
COMPOSITIONS DE FIXATEUR ET PROCÉDÉS DE CONSERVATION D'ÉCHANTILLONS BIOLOGIQUES

(30) Priority: 28.10.2020 US 202063106422 P
(43) Date of publication of application: 06.09.2023
(73) Proprietor: University Health Network, Toronto, Ontario M5G 2C4 (CA)
(72) Inventor: MURPHY, Kieran, Toronto, Ontario M4V 2L5 (CA); VUCEVIC, Diana, Toronto, Ontario M9R 1B1 (CA); KESHAVJEE, Shafique, Toronto, Ontario M4N 3C6 (CA)
(74) Representative: Greaves Brewster LLP
(86) International application number: PCT/CA2021/051526
(87) International publication number: WO 2022/087741

(56) References cited:
- LAVINGIA B: "Honey dextran medium in preservation of cornea", INDIAN JOURNAL OF OPHTHALMOLOGY, ALL-INDIA OPHTHALMOLOGICAL SOCIETY, BOMBAY, IN, vol. 31, no. 5, 30 November 1982 (1982-11-30), pages 671 - 673, XP009536839, ISSN: 0301-4738
- LALWANI VIDUSHI ET AL: "Honey as an alternative fixative for oral tissue:An evaluation of processed and unprocessed honey", JOURNAL OF ORAL AND MAXILLOFACIAL PATHOLOGY : JOMFP, vol. 19, no. 3, 1 January 2015 (2015-01-01), IN, pages 342 - 347, XP055934750, ISSN: 0973-029X, DOI: 10.4103/0973-029X.174641
- KESHAVJEE S.H. ET AL: "The role of dextran 40 and potassium in extended hypothermic lung preservation for transplantation", THE JOURNAL OF THORACIC AND CARDIOVASCULAR SURGERY, vol. 103, no. 2, 1 February 1992 (1992-02-01), US, pages 314 - 325, XP055934749, ISSN: 0022-5223, DOI: 10.1016/S0022-5223(19)35033-0
- FISCHER STEFAN ET AL: "Raffinose improves 24-hour lung preservation in low potassium dextran glucose solution: a histologic and ultrastructural analysis", THE ANNALS OF THORACIC SURGERY, vol. 71, no. 4, 1 April 2001 (2001-04-01), pages 1140 - 1145, XP093191305, ISSN: 0003-4975, DOI: 10.1016/S0003-4975(01)02426-2
- VUCEVIC DIANA D ET AL: "A Novel Tissue Preservation and Transport Solution as a Substitute for Formalin.", LABORATORY INVESTIGATION; A JOURNAL OF TECHNICAL METHODS AND PATHOLOGY 09 2023, vol. 103, no. 9, September 2023 (2023-09-01), pages 100198, XP002811973, ISSN: 1530-0307
- LAVINGIA B: "Honey dextran medium in preservation of cornea", INDIAN JOURNAL OF OPHTHALMOLOGY, vol. 31, no. 5, 30 November 1982 (1982-11-30), IN , pages 671 - 673, XP009536839, ISSN: 0301-4738
- KESHAVJEE S.H., YAMAZAKI F., YOKOMISE H., CARDOSO P.F., MULLEN J.B.M., SLUTSKY A.S., PATTERSON G.A.: "The role of dextran 40 and potassium in extended hypothermic lung preservation for transplantation", J THORAC CARDIOVASC SURG, 1992, pages 314 - 325, XP055934749
- LALWANI VIDUSHI, SUREKHA R, VANISHREE M, KONERU ANILA, HUNASGI SANTOSH, RAVIKUMAR SHAMALA: "Honey as an alternative fixative for oral tissue: An evaluation of processed and unprocessed honey", J ORAL MAXILLOFAC PATHOL, vol. 19, no. 3, September 2015 (2015-09-01), pages 342 - 347, XP055934750
- "Bancroft's theory and practice of histological techniques, 8th ed.", 30 November 2018, ELSEVIER, ISBN: 978-0-7020-6864-5, article CHRISTOPHER LAYTON, JOHN D. BANCROFT, S. KIM SUVARNA: "4 - Fixation of tissues", pages: 40 - 63, XP009537182, DOI: 10.1016/B978-0-7020-6864-5.00004-9

## Description

This Patent Cooperation Treaty application claims the benefit of priority of United States Provisional Application 63/106,422 filed October 28, 2020.

### FIELD

The present disclosure relates to compositions for fixing tissue and particularly for fixing tissue while maintaining DNA and/or RNA integrity in tissue or cells for example during its storage or where later analysis of DNA or RNA may be desirable, and methods of making such compositions.

### Background

The current clinical practice of tissue biopsy transport storage is a Formalin-fixed paraffin-embedded (FFPE) tissue block that stabilizes tissue through cross-linking macromolecules. Formalin (-CH2O) is made from 40% formaldehyde. Formalin is a common diagnostic routine used in almost all pathology laboratories [1]. Pathologists are trained on the nuclear staining of formalin for histopathological analysis. However, formalin is classified as a carcinogenic and toxic solution [2]. Evaporation from fume hoods, transport handling and spilling of formalin all increase clinical practitioners' exposure to the human carcinogen that has been linked to nasopharyngeal cancer reported by The International Agency for Research of Cancer [3]. Several European countries have banned the use of formaldehyde [3]-[4] making alternatives very critical to the health care sector. Multiple studies have shown correlation of formalin on tissue DNA degradation through chemical modification, DNA trapping and fragmentation [5]-[6]. RNA tends to significantly degrade in formalin fixed samples. This has been attributed to poly-A tail damage and covalent mutation of RNA nucleotide bases by the addition of monomethylol -CH2OH [7]. A problem with the FFPE design is that it is only able to preserve cellular proteins for immunohistochemistry type tests [6]. It is not designed to maintain the integrity of the DNA or RNA yield of the tissue sample. The extraction of nucleic acids from a FFPE tissue block becomes very challenging because of the low quantity and quality left due to degradation. The integrity of DNA/RNA yield is important for tumor biopsies to achieve accurate molecular analysis.

The funeral sector relies heavily on formaldehyde as part of an embalming fluid. About 20 million litres of embalming fluid are used in the U.S alone. The purpose of an embalming fluid is to delay the body's rate of decomposition - fixing the bodies of the deceased and trying to maintain a natural appearance for open casket viewings. In addition to the health hazards formaldehyde poses to workers in exposure, the toxicity of formaldehyde leaches into the air and soil with each burial.

Fixative Formalin is the common clinical practice for fixative tissue biopsies. Other methods and compositions have been used.

### Liquid nitrogen

The goal of freezing tissue specimen is to have a short time frame from the biopsy procedure to the freezing storage. However, the clinical practicality of having liquid nitrogen in the operating room is not feasible. The biopsy samples will arrive to the pathologist who will dissect the clinical samples for cryogenic storage. The transport from procedure to pathologist of the tissue biopsy sample can be up to 10 hours. This time frame is crucial for cells to be kept stable in a fixative solution. The use of liquid nitrogen to preserve tissue biopsy specimen is commonly seen for lung tumors due to the difficulty of obtaining a high quantity RNA yield for post microarray analysis [8]. Biopsy samples are snap frozen in liquid nitrogen and maintained at -80 degrees Celsius until pathological and molecular evaluation. Samples are thawed and centrifuged at room temperature for RNA extraction [8]. A limitation with this method is snap freezing can be cost-intensive and limited to certain laboratories and universities with access to equipment.

### RNAlater and AllProtect

Two solutions commercially developed by Qiagen, RNAlater^{™} and AllProtect^{™}, are utilized as tissue preservation solutions where RNA and DNA are protected. RNAlater is composed of a sulphate salt solution in which its controlled pH helps precipitate RNases and other soluble proteins and stabilizes RNA quality [9]. Allprotect works by engulfing the tissue as a protective layer to prevent from degradation [9]. Allprotect provides rapid stabilization of DNA, RNA and protein in tissue specimen at room temperature. Both solutions replace the need for liquid nitrogen or dry ice. These two solutions can be expensive for mass clinical practice.

### Methacarn and RCL2

Methacarn and RCL2 were developed as noncrosslinkning fixatives as an alternative to formalin fixative. A study done by Delfour and others showed that the histomorphology of methacarn and RCL2 fixed paraffin-embedded breast tumors was similar to FFPE tissues with respect to cytoplasmic and nuclear information [10]. Methacarn was shown to "maintain tissue morphology and to preserve DNA, RNA and protein integrity" [10]. It is composed of 60%(v/v) methanol, 30% chloroform and 10% glacial acetic acid. RCL2, composed of acetic acid and ethanol [11], is a promising fixative because of its ability to preserve nucleic acid and protein [3][10]. The study fixed breast tumor biopsy in methacarn or RCL2 (10ml per cell pellet) at 4 degrees Celsius overnight. Samples were then dehydrated in ethanol and acetone and embedded in paraffin for 1 hour at 58 degrees Celsius. Both fixatives proved to preserve DNA integrity by yielding high quantity of DNA extraction and amplification of DNA sequences [10][12]. In contrast, formalin-fixed tissues have shown high amounts of nonreproducible fragments, resulting with "1 mutation artifact per 500 bases" [10]. These two solutions however also contain toxic elements in their composition.

### Ethanol

Ethanol was historically used a fixative before the introduction of formalin [12]. A study done by Sarot and others in Berlin compared 3 fixatives; formalin, pure ethanol and RCL2 on euthanized rats. Excision of abdominal skin (1 cm2) and tail (1 cm) was used for sampling and stored at -80 degrees. The study demonstrated that formalin fixed samples had a loss of DNA extraction yield while the alcohol-based fixatives remained unvaried [11]. It was proven both ethanol and RCL2 have better performance than formalin for both fixation samples and kinetic preservation up to 24 hours at temperatures of 55 degrees Celsius and 75 degrees Celsius. The study summarized all three fixatives by concluding, "Formalin remains the best fixative for maintaining the morphological integrity, RCL2 appears as the best tested fixatives for DNA assessment and pure ethanol would be the best compromise to manage whole parameters" [11].

### Others

A study done by Lalwani et al tested oral tissue fixed in processed and unprocessed honey against 10% formalin [13]. Honey was prepared in a 1:10 ratio with distillated water. Results showed that for nuclear staining, all three solutions showed 100% fixing and staining coherence. With regards to tissue morphology, the study showed 72% adequacy for both honey solutions and 92% adequacy for 10% formalin. A study done by Rajanikanth et al, showed that the staining quality, cellular outline, nuclear details, and tissue foldings using honey or coconut oil scored high in each criterion [14].

Solutions are known for organ preservation and perfusion.

### Perfusion and Organ Preservation solutions

There are several tissue-organ transplantation preservation and perfusion solutions currently used in clinical practice: Histidine-tryptophan-ketoglutarate (HTK), University of Wisconsin (UW), St. Thomas solution, Euro-Collins solution (EC), and Kurt-Ozcan (KO) [15] being of the most common. Table 1 illustrates the components of each preservation solution.

Histidine-tryptophan-ketoglutarate (HTK), is a low potassium solution used both as perfusion and preservation solution of liver, kidney, heart, lung and pancreas donor organs [16]. University of Wisconsin (UW) is an intracellular preservation medium for organ transplantation and remains the gold standard for organ transplantation. It is a cardioplegic solution used to facilitate perseveration of liver, pancreas and kidney transplants [17]. St Thomas is a common extracellular cardioplegia solution is primarily used for donor heart preservation [17]. Euro-Collins is an intracellular solution based on high potassium, low sodium and high concentration of glucose. Euro-Collins (EC) is a high potassium solution used for pulmonary artery perfusion for the preservation of lung and kidney transplants [9]. Kurt-Ozcan (KO) was developed for skeletal muscle biopsy samples to protect the morphological, enzyme histochemical, biochemical, and molecular characteristics as an alternative to liquid nitrogen method [15]. In addition to this list, a lung perfusion extracellular solution called Perfedax incorporates low K+, high Na+ and Dextran [18]. Lavingia B., Honey Dextran medium in preservation of cornea. Indian J Ophthalmol. 1983 Sep;31(5):671-3 discloses a preservation medium comprising honey and dextran, in particular a honey-dextran composition used for maintaining corneal tissue, wherein the eye tissue is perfused with a dextran or dextran/Ringer's solution prior to storage.

**Table 1: Comparison of Different Perfusion and Preservation Solutions**

| *Components (Mmol*/*L) HTK* | | *UW* | *St.Thomas* | *EC* | *KO* | *LPDG* |
|---|---|---|---|---|---|---|
| *Na* | 15 | 28 | 120 | 10 | 0 | 138 |
| *K* | 9 | 125 | 16 | 115 | 0 | 6 |
| *Cl* | 32 | 0 | 0 | 15 | 0 | 142 |
| *Mg* | 4 | 0 | 16 | 0 | 0 | 0.8 |
| *SO4* | 0 | 4 | 0 | 0 | 0 | 0.8 |
| *PO4* | 0 | 25 | 0 | 57.5 | 0 | 0.8 |
| *Ca* | 0.015 | 0 | 1.2 | 0 | 0 | 0.3 |
| *HC03* | 0 | 5 | 10 | 10 | 0 | 1 |
| *Dextran 40* | 0 | 0 | 0 | 0 | 0 | 50 |
| *Glucose* | 0 | 0 | 0 | 3.5 | 0 | 0.9 |
| *Raffinose* | 0 | 30 | 0 | 0 | 30 | 0 |
| *Lactobionate* | 0 | 100 | 0 | 0 | 100 | 0 |
| *Glutathione* | 0 | 3 | 0 | 0 | 3 | 0 |
| *Adenosine* | 0 | 5 | 0 | 0 | 5 | 0 |
| *Allopurinol* | 0 | 1 | 0 | 0 | 1 | 0 |
| *Pentafraction* | 0 | 50 | 0 | 0 | 0 | 0 |
| *Histidine* | 198 | 0 | 0 | 0 | 0 | 0 |
| *Mannitol* | 38 | 0 | 0 | 0 | 0 | 0 |
| *MgSO4* | 0 | 0 | 0 | 0 | 5 | 0 |
| *KOH* | 0 | 0 | 0 | 0 | 25 | 0 |
| *KH2PO4* | 0 | 0 | 0 | 0 | 25 | 0 |
| *Ketoglutarate* | 1 | 0 | 0 | 0 | 0 | 0 |
| *Tryptophan* | 2 | 0 | 0 | 0 | 0 | 0 |

Fixative solutions that maintain the integrity of proteins and nucleic acids are desirable.

### SUMMARY

Provided herein are compositions that can be used for fixing tissue biopsies (e.g. fixative compositions) and that maintains DNA and/or RNA integrity during storage and/or transport for histopathological analysis. The compositions provided are an alternative to formalin, and for example improve the preservation of DNA and/or RNA integrity and yield of tissue biopsies compared to formalin. Further, various formulations described allow for increased magnetic resonance imaging (MRI) T1 signal reducing for example imaging artifacts resulting from the tissue-air interface, for example in MRI, and may therefore allow for more accurate imaging.

In preliminary testing, the formulations identified as "Amber" which refers to honey based formulations with dextran and optionally coconut oil, has been shown to have better preservation of rat tissue (e.g. structural preservation of proteins, carbohydrates, and other bio-active moieties in their spatial relationship to the cell, also preservation of DNA/RNA integrity) than formalin. Tissue samples in Amber formulations after 24 hours both at room temperature and 4 degrees, are pink and red - resembling fresh tissue. While tissue stored in formalin after 24 hours was grey and stiff. The liquid compositions described herein may be suitable, for example as a tissue transport medium or tissue fixative, particularly where both cell morphology and nucleic acid based analyses are of interest. It can also be used as an embalming fluid formulation or to make an embalming fluid formulation for example as a replacement for formaldehyde, glutaraldehyde, methanol and other solvents.

The fixative compositions of the present invention are defined in claim 1. Formulations comprising coconut oil, may be particularly useful for overcoming tissue air interface artifacts and for increasing MRI T1 signal.

Another aspect is a method of making a biological sample fixative composition as defined in claim 9.

Another aspect is a method of preserving a biological sample as defined in claim 11.

A further aspect (not claimed) is use for biological samples that are to be assayed, for example by immunohistochemistry or other immune based assays. In some embodiments, the biological samples are assayed or further assayed for molecular analyses such as in situ hybridization assays, PCR based assays etc. In some embodiments, the biological samples are assayed for example, by one or more of immunofluorescence analysis, histological examination, and/or a nucleic acid analysis, optionally DNA extraction and/or PCR analysis.

Accordingly, also provided herein are uses of compositions described herein for fixing biological samples that are used for immunohistochemistry or immune based methods and/or for molecular analyses for example those involving personalized medicine.

The biological sample can be any cells or tissue including cancer cells and tissues, for example lung cancer or breast cancer cells or tissue.

A further aspect of the invention is a kit as defined in claim 15.

### BRIEF DESCRIPTION OF THE DRAWINGS

An embodiment of the present disclosure will now be described in relation to the drawings in which:
Fig. 1 is an image of a pathologist box with a tissue specimen in Amber solution for imaging.
Fig. 2A Histology H&E staining 20x and 100x oil magnification. Formalin (left) and Amber (right) rat tissue 24 hour storage at 4 degrees Celcius. B Mann-Whitney non-parametric test of histology scoring 0-3. Formalin shows a median of 2 and Amber a median 3.

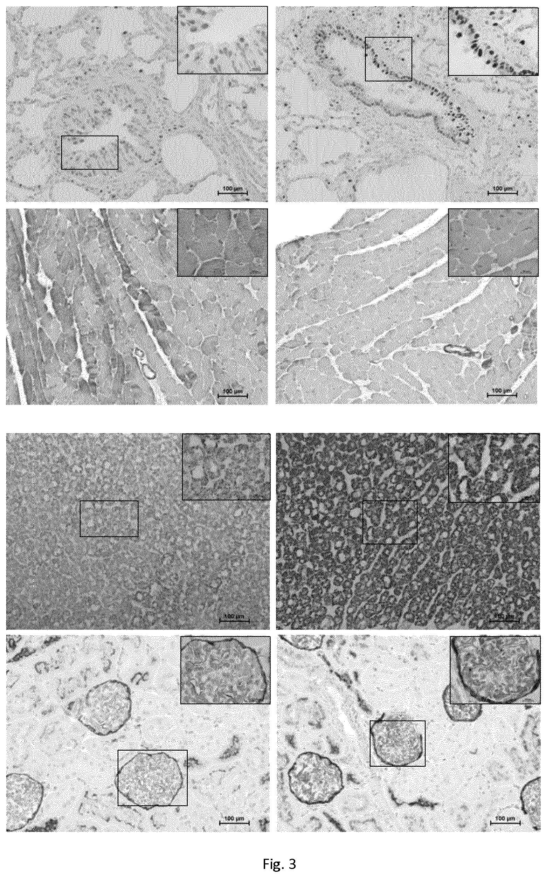

Fig. 3 IHC at 20x magnification and 100x oil with 4 representative antibodies: Lung: Thyroid Transcription Factor (TTF-1), Heart: Muscle Specific Actin (MSA), Liver: Hepatocyte Paraffin 1 (HepPar1) and Kidney: Common Acute LymphoblasticCD10.
Fig. 4 IF staining VE-Cadherin, CY3, exposure time 500ms. A Formalin and B Amber paraffin embedded. All taken at 20x magnification.
Fig. 5 IF staining of Vimentin (VIM), CY3 exposure time 300ms and Alpha Smooth Muscle Actin (SMA), GRP exposure time 300ms A Lung tissue B Liver tissue C Kidney tissue D Heart tissue paraffin embedded. All taken at 20x magnification.
Fig. 6 RNA extraction. Rat lung tissue in 24-hour storage at 4 degrees in Amber, PBS and formalin. After storage, tissue was ready for RNA extraction following the Trizol method.

### DETAILED DESCRIPTION OF THE DISCLOSURE

In understanding the scope of the present disclosure, the term "comprising" and its derivatives, as used herein, are intended to be open ended terms that specify the presence of the stated features, elements, components, groups, integers, and/or steps, but do not exclude the presence of other unstated features, elements, components, groups, integers and/or steps. The foregoing also applies to words having similar meanings such as the terms, "including", "having" and their derivatives.

The term "honey", as used herein, refers to honeys originating from several sources, for example Manuka honey, alfalfa honey, eucalyptus honey, acacia honey, buckwheat, and having a viscosity of at least 10,000 cP at room temperature. Viscosity of honey can range depending on the type 10,000cP-12,000cP. Exemplary honeys are provided in Table 3.

The term "dextran" as used herein refers to a branched glucan composed of linear α(1→6) linked glucose units and α (1→3) link initiated branches. Dextran ranges in size from 10,000 to 150,000 Kd. The dextran may for example be about 40, 000 Kd (e.g. size range 40,000 Kd +/-10%). For example, Dextran 40^{™} is a mixture of glucose polymers that are about 40,000 Kd. Dextran 70 can also be used. The dextran can be provided for example using a dextran containing composition, such as a low potassium dextran (LPDG) solution.

The term "lactobionate" as used herein, refers to a sugar acid, specifically, the carboxylate anion of lactobionic acid, a disaccharide formed form gluconic acid and galatactose.

As used herein "a biological sample" means any sample from a subject such as a human and comprising, cells and/or tissue, typically containing DNA and/or RNA, optionally the sample comprising cancer cells , for example tumor cells, including for example a tumor tissue sample such as a biopsy, tissue slice, or cancer cells.

The term "biopsy" as used in herein includes all types of biopsies known to those skilled in the art. Thus, the term "biopsy", as used in the context of the present invention, may include, for example, samples obtained by resection of tumors, tissue samples obtained by endoscopic methods, or organ biopsies obtained using forceps or a needle, e.g. fine need biopsies, for example, for histopathological analysis and the like.

The term "integrity" as used herein with respect to nucleic acids, refers to the state or degree of degradation or lack thereof. A biological sample with nucleic acid integrity implies only up to a minimal level of degradation, for example, where DNA or RNA in a biological sample has experienced less than 30% degradation, less than 20% degradation or less than 10% degradation, for example, when measured using a nucleic acid assay such 3'/5' assay for analysis of RNA integrity and/or has a 260/280 ratio of greater than 1.7 The expected A260/A280 of double-stranded DNA is 1.7-1.9. For RNA it is 1.8-2.0. Ratios close to 1.8 2.0 are considered to be intact or have very high integrity.

The term "pathologist box" as used herein, refers to a transparent box under which one can view a sample under imaging. A pathologist's box is a container for the specimen that has no artifact on medical imaging and is compatible with all imaging yet it allows safe and secure transport of the pathology specimen, for example in Amber formulations or other formulations described herein.

The terms "preservation" or "fixation" as used herein interchangeably refer to for example a state wherein the native appearance and intact state of the sample is prolonged or better prolonged compared to the absence of preservation or fixation. For example, where the biological sample is a resected biological sample, optionally tissue, preservation or fixation may maintain nucleic acid integrity, and/or structural or morphological integrity so it more resembles fresh resected sample, or sample in vivo. Preservation and fixation can also be understood as a state of delaying decomposition of the sample from its in vivo or natural state.

The terms "fixing" or "preserving" as used herein interchangeably refer to subjecting a biological sample, optionally tissue, to a fixative composition and/or series of steps including subjecting said biological sample to said fixative composition, for sufficient time and under conditions that results in sample preservation.

The term "consisting" and its derivatives, as used herein, are intended to be closed ended terms that specify the presence of stated features, elements, components, groups, integers, and/or steps, and also exclude the presence of other unstated features, elements, components, groups, integers and/or steps.

The term "composition" as used herein, a mixture comprising two or more compounds. In an embodiment a composition is a composition of two or more distinct compounds. In a further embodiment, a composition can comprise two or more "forms" of the compounds, such as, salts, solvates, or, where applicable, stereoisomers of the compound in any ratio. A person of skill in the art would understand that a compound in a composition can also exist as a mixture of forms. For example, a compound may exist as a hydrate of a salt. All forms of the compounds disclosed herein are within the scope of the present disclosure.

Further, terms of degree such as "substantially", "about" and "approximately" as used herein mean a reasonable amount of deviation of the modified term such that the end result is not significantly changed. These terms of degree should be construed as including a deviation of at least ±5%, optionally ±10%, and/or up to ±25% of the modified term if this deviation would not negate the meaning of the word it modifies.

As used in this specification and the appended claims, the singular forms "a", "an" and "the" include plural references unless the content clearly dictates otherwise.

The definitions and embodiments described in particular sections are intended to be applicable to other embodiments herein described for which they are suitable as would be understood by a person skilled in the art.

The recitation of numerical ranges by endpoints herein includes all numbers and fractions subsumed within that range (e.g. 1 to 5 includes 1, 1.5, 2, 2.75, 3, 3.90, 4, and 5). It is also to be understood that all numbers and fractions thereof are presumed to be modified by the term "about".

Further, the definitions and embodiments described in particular sections are intended to be applicable to other embodiments herein described for which they are suitable as would be understood by a person skilled in the art. For example, in the following passages, different aspects of the disclosure are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

In some embodiments, the fixative composition comprises at least 0.5 percent to 15 percent coconut oil, preferably, at least 1 percent coconut oil. In an embodiment, the coconut oil is less than 10% of the final composition v/v. Coconut oil can be included, particularly when used for tissues that will be imaged, such as by MRI, producing for example a slightly bright T1 weighted signal. Range can be about 0%-1.5% coconut oil of the final composition v/v.

According to the present invention, the syrup is honey. The fixative solution comprises honey and dextran. In some embodiments, the honey is pure honey or raw honey.

In one embodiment, the honey is a honey described in Table 3 or a combination thereof. In a further embodiment, the honey in the honey-based solution has a viscosity of about or at least 10,000 cP at room temperature. In another embodiment, the coconut oil has a viscosity of 60 cP at room temperature.

The fixative composition further comprises sodium. The composition comprises about 80mmol/L to about 190mmol/L, or any number including or in between 80mmol/L to about 190mmol/L, for example a preferred concentration is 138mmol/L.

The fixative composition further comprises potassium. The composition comprises about 1mmol/L to about 15 mmol/L, or any number including or in between 1mmol/L to about 15 mmol/L for example about 6mmol/L of potassium (e.g. about 6mmol of potassium per litre of composition solution).

The fixative composition further comprises chlorine. The composition comprises about 50mmol/L to about 150mmol/L, or any number including or in between 50mmol/L to about 150mmol/L for example about 142mmol/L of chlorine.

The fixative composition further comprises magnesium. The composition comprises about 0.1mmol/L to about 3 mmol/L, or any number including or in between 0.1mmol/L to about 3 mmol/L, for example about 0.8mmol/L of magnesium.

The fixative composition further comprises sulfate. The composition comprises about 0.1mmol/L to about 3 mmol/L, or any number including or in between 0.1mmol/L to about 3 mmol/L, for example about 0.8mmol/L of sulfate.

The fixative composition further comprises phosphate. The composition comprises about 0.1mmol/L to about 3 mmol/L, or any number including or in between 0.1mmol/L to about 3 mmol/L, for example about 0.8mmol/L of phosphate.

The fixative composition further comprises calcium. The composition comprises about 0.1mmol/L to about 3 mmol/L, or any number including or in between 0.1mmol/L to about 3 mmol/L for example about 0.3mmol/L of calcium.

The fixative composition further comprises bicarbonate. The composition comprises about 0.1mmol/L to about 15mmol/L, or any number including or in between 0.1mmol/L to about 15mmol/L, for example about 1 mmol/L of bicarbonate.

The fixative composition comprises dextran. The composition comprises about 10g/L to about 60g/L, or any number including or in between10g/L to about 60g/L, for example about 50g/L of dextran. In a further embodiment, the dextran is Dextran 40^{™}. In another embodiment, the Dextran is Dextran 70 ^{™}. The dextran can be provided using a low potassium solution, e.g. LPDG. In some embodiments, the dextran or LPDG is provided by for example purchased solutions.

In another embodiment, the fixative composition further comprises a simple sugar such as glucose. In a further embodiment, the composition comprises about 0.1g/L to about 5g/L, or any number including or in between about 0.1g/L to about 5g/L, for example about 0.9 g/L of glucose. Another simple sugar that could be used is fructose.

In another embodiment, the fixative composition further comprises a trisaccharide. In a further embodiment, the composition comprises about 0.1mmol/L to about 50mmol/L or any number including or in between 0.1mmol/L to about 50mmol/L, for example about 30 mmol/L of the trisaccharide. In a further embodiment, the trisaccharide is raffinose. Raffinose has been useful can be useful for controlling cell tonicity, for example, maintaining cell hypertonicity.

In another embodiment, the fixative composition solution further comprises a sugar acid. In a further embodiment, the composition comprises up to about 100mmol/L, for example or any number including or in between 0.1mmol/L to about100mmol/L of the sugar acid. In a further embodiment, the sugar acid is lactobionic acid or the gluconate thereof, lactobionate. The sugar acid can be used for example as an excipient for formulation, or to aid in long-term stabilization of the composition.

Potassium lactobionate can also be used.

In another embodiment, the fixative composition further comprises a free radical scavenger. In a further embodiment, the composition comprises up to about 10mmol/L, or any number including or in between about 0.1 mmol/L and about 10 mmol/L, for example about 3mmol/L of the free radical scavenger. In a further embodiment, the free radical scavenger is glutathione. Glutathione for example, hinders damage to cell from free radicals, peroxides, lipid peroxides, and heavy metals. α-tocopherol, ascorbic acid, β carotene, selenium can alternatively be used.

For ranges described herein, subranges are also contemplated, for example every, 0.1 increment there between. For example, if the range is 100mmol/L to about 150mmol/L, also contemplated are 100.1 mmol/L to about 150mmol/L, 100mmol/L to about 149.9mmol/L, 100.1 mmol/L to about 149.9 mmol/L and the like.

In another embodiment, the fixative composition, wherein the concentration of one or more of the components of the composition is provided in Tables 2 and/or 6. In a further embodiment, the fixative composition comprises the components and their concentrations as provided in Table 2 and/or 6.

The fixative composition can also comprise a RNAse inhibitor and/or a DNAse inhibitor.

The fixative composition can comprise a pH of about 3 to about 10.5, and includes for example a fixative composition with a pH of about 3.5, 4, 4.5, 5, 5.5, 6, 6.5, 7, 7.4, 7.5, 8, 8.5, 9, 9.5 or about 10. In some embodiments, the fixative composition has a pH from about 7 to about 7.5. In some embodiments, the pH is or is about 7.4.

In some embodiments, the sample is immersed into the fixative composition. In other embodiments, the fixative composition is poured onto the sample. In yet other embodiments, the fixative solution is injected into the sample. A combination of methods can also be used.

The biological sample can be any cell source or tissue, is particularly useful for samples wherein the preservation of DNA and/or RNA integrity and protein or cell morphology or structure is desired. In an embodiment, the biological sample is or comprises cancer cells. In a further embodiment, the biological sample is a tissue sample, optionally a biopsy, including for example a fine needle biopsy or a surgical specimen. In some embodiments, the biological sample is lung, heart, liver, or kidney tissue. The biological sample is not particularly limited.

As demonstrated herein, the fixative composition can preserve DNA and/or RNA integrity as well as morphological or structural features in the biological sample, for example similar to or better than formalin. As demonstrated herein, the fixative compositions described herein comprising for example honey, dextran and coconut oil, permit isolation of a high quantity of RNA (see for example Table 7). As shown, formalin does not permit recovery of intact RNA (as indicated by RIN of N/A).

In an embodiment, the composition is a liquid formulation. The composition can also be a dried or dehydrated formulation that is reconstituted for example with water or other diluent. Reconstitution or rehydration can take place when needed or desired.

The fixative composition can be used to preserve the biological sample comprising DNA and/or RNA integrity for at least 8 hours, at least 10 hours, at least 12 hours, at least 14 hours or at least or up to 48 hours or longer, optionally at about 4C. The fixative composition can also be used to preserve such samples for longer periods of time or be combined for example with freezing for example in liquid nitrogen. In some embodiments, the composition, is cooled down to about 4 degrees Celsius prior to use. In other embodiments the composition is at room temperature. The composition can for example be any temperature. Liquid formulations are typically kept above freezing to about 25C, although they can also be frozen. As described herein, the fixative compositions can be used for preserving biological samples, optionally tissue.

In another embodiment, the fixative composition is a formulation that increases MRI T1 signal of a biological sample subjected to MRI. The development of imaging technology such as Optical Coherence Tomography (OCT) for resected tissue biopsies allows for tumor margin evaluation. A challenge with imaging resected tissue is the air around the specimen. The tissue-air interface creates artifacts on the imaging that can lead to misrepresentation of the diagnosis. Even beyond its fixative properties, Amber overcomes the tissue-air interface by suspending the tissue in coconut oil, particularly valuable for increasing MRI T1 signal. For example, the composition can comprise coconut oil as described herein. Alternatively, the fixative composition can comprise canola oil, vegetable oil, olive oil or glycerol.

Also provided are methods of making the above compositions, for example combining the components of the aforementioned composition. The syrup such as honey may be diluted for example in water or LPDG, having a temperature of less than 160°F, optionally less than 140°F and greater than for example 100°F. The water may be distilled water. Alternatively the syrup, optionally honey, and/or coconut oil can be heated and added to diluent such as a dextran comprising solution such as LPDG. Other components may be added to the diluted syrup according to standard formulation practices. For example techniques for formulation are described in "Remington's Pharmaceutical Sciences", 18th Edition, Mac Publishing, Inc. Publishing Co. ) [Easton, Pa., USA] (1990).

The solutions can be prepared by first adding syrup to a dextran comprising solution and then adding coconut oil if coconut oil is to be added. The order of mixing is not particular limited. Syrup such as honey, and/or coconut oil may be added last and/or second last to a liquid formulation.

The pH can be adjusted with any pH adjusting agent or neutralizing agent. For example, in formulations where the pH is acid, a caustic agent, such as NaOH or diluted NaOH can be used to adjust the pH, for example to about 7.4. Sodium carbonate can also be used.

As mentioned, the solution can be dehydrated for later reconstitution, for example with distilled water.

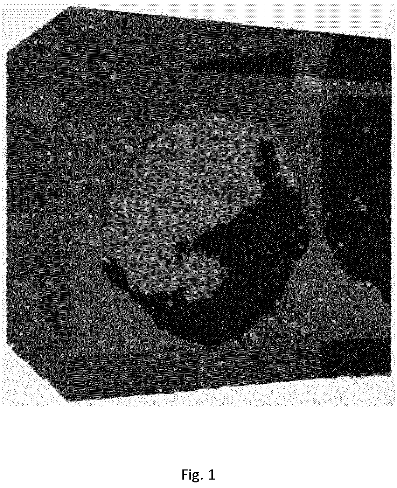

Also provided are uses of the above composition, for example preserving a biological sample using the aforementioned composition, optionally by immersing the biological sample in the aforementioned solution preferably immediately after the excision of a biological sample, optionally adding the biological sample to the composition, for example in a container as shown in Figure 1 or into a box for imaging, or adding the composition the biological sample, where the sample is placed in a multiwall plate. In another embodiment, the biological sample is fixed for imaging such as MRI viewing, cell based and/or nucleic based assays or where the tissue is in a corpse (e.g. embalming).

For example, a biological sample that has been incubated with a fixative solution described herein, for example for at least 8 hours, 16 hours, 24 hours or 48 hours, can be processed similar to formalin fixed tissue. For example, the fixed sample can be dehydrated with ethanol, isopropanol and/or xylene and infused and/or encased in paraffin or wax and subjected to analysis as described for example in Examples 3 and 4, and including histology, IHC or DNA/RNA extraction, immunofluorescence analysis, histological examination, and/or a nucleic acid analysis, optionally DNA extraction and/or PCR analysis.

In particular, the fixative compositions described herein are useful when multiple analyses may be performed, such as IHC or imaging, followed by molecular analysis for personalizing care. The molecular analysis can be a mutational assessment and can comprise extracting DNA or RNA and detecting the presence or absence of the mutation.

For example, common DNA/RNA extraction kits can be used with samples fixed using the fixative compositions described herein. Examples for RNA extraction include, RNeasy FFPE Kit and for DNA include Gene Read DNA FFPE Kit. Spectrophotometric methods (e.g. UV absorbance) can also be used.

The formulations can be made using known formulation techniques. Making the formulation can include for example dissolving the syrup such as honey in a low potassium dextran (LPDG) solution by heating for example in a microwave briefly. 1part syrup (e.g. honey) can be combined with 9 parts LPDG. Coconut oil can also be added for example to provide about 1% coconut oil in the final solution. Dispersing of the components can be achieved by mixing. Honey may be dissolved in LPDG without the addition of coconut oil. Further, depending on the formulation, the syrup such as honey can be dissolved in distilled water and coconut oil added for example to provide a final concentration of coconut oil of 1%.

Another aspect includes a container comprising the aforementioned fixative composition. In an embodiment, the container further comprises a biological sample to be fixed. Such containers comprising sample can be used to ship samples to different locations. In another embodiment, the container and/or the composition is sterile. In a further embodiment, the container is labelled. In another embodiment, the container is a pathologist's box.

A further aspect includes a kit comprising a container comprising the aforementioned composition. In an embodiment, the container is sterile. In another embodiment, the kit comprises one or more containers, wherein at least one container is pre-filled with the aforementioned fixative composition. In a further embodiment, the kit further comprises an instrument for obtaining biopsies. In another embodiment, at least one of the one or more containers is a pathologist box. The kit can comprise a standard tissue biopsy transport container which is about the size of a yogurt container.

The fixative compositions can be used to fix DNA and/or RNA in the tumor tissue. The fixative compositions can also be used to aid in personalized vaccine development. For example, a tumor sample can be fixed with a fixative solution described herein. Subsequently, the tumor nucleic acids can be sequenced to obtain information on their sequence compositions with the aim to develop personalized vaccines for cancer patients which trigger particular mutations. For example, in Glioblastoma multiforme patient vaccines personalized to the patient are developed. These have shown better survival than conventional therapy. Amber will allow better preservation of DNA and RNA greater diversity and stability of epitopes be available for vaccine development. Accordingly, the fixative solutions can be included in kits and/or used for tumor genetic mutation assessment.

The above disclosure generally describes the present application. A more complete understanding can be obtained by reference to the following specific examples. These examples are described solely for the purpose of illustration and are not intended to limit the scope of the application. Changes in form are contemplated as circumstances might suggest or render expedient. Although specific terms have been employed herein, such terms are intended in a descriptive sense and not for purposes of limitation.

The following non-limiting examples are illustrative of the present disclosure:

### EXAMPLES

### Example 1

Some of the Amber formulations incorporate low potassium (e.g. at a concentration of about 6 +/-5 mmol) and/or high sodium (e.g. at a concentration of about 138 +/- 50 mmol), content which can limit reactive oxygen species, lactobionate which is a sugar acid that provides for example an excitant for the formulation, glutathione which can hinder damage to cells from free radicals, peroxides, lipid peroxides and heavy metals, honey. It is found herein, that for preserving DNA and RNA integrity and fixation of the sample, honey in a 1:10 dilution can be used. In addition, coconut oil is used in some formulations to provide a lipid for imaging properties on MRI. Coconut oil has similar properties as honey with regards to the preservation of tissue. Exemplary composition includes the following components listed in table 2. Several types of honey listed in table 3 are being tested.

**Table 2. Composition of Solution - Amber.**

| *Components* | *Concentrations(mmol*/*L)* |
|---|---|
| *Na* | 138 |
| *K* | 6 |
| *Cl* | 142 |
| *Mg* | 0.8 |
| *SO4* | 0.8 |
| *PO4* | 0.8 |
| *Ca* | 0.3 |
| *HCO3* | 1 |
| *Dextran 40* | 50 (g/L) |
| *Glucose* | 0.9 (g/L) |
| *Raffinose* | 30 |
| *Lactobionate* | 100 |
| *Glutathione* | 3 |
| *Honey 10%* | 10% |
| *Coconut Oil 1%* | 1% |

**Table 3. Different Types of Honey and Benefits.**

| | |
|---|---|
| **Type of Honey Manuka** | **Reason** |
| | -High concentrations of methylglyoxal (MGO) and dihydroxyacetone |
| | -antibacterial activity [25] |
| | -Vitamins B1,B2,B3,B5 and B6 |
| | -Amino acids: lysine, proline, arginine and tyrosine |
| | -Minerals: calcium, magnesium, copper, potassium, zinc and sodium [26] |
| **Alfalfa** | -Contains fructo oligosaccharides □ Prebiotic effects [26] - Antipyretic agent |
| **Eucalyptus** | -Contains luteolin, kaempferol, quercetin, myricetin and ellagic acid □ antioxidant and anti-inflammatory agent [27] |
| | -Minerals: sodium, potassium, manganese, magnesium, iron, copper and zinc |
| **Acacia** | -Hepatoprotective and nephroprotective effects [28] |
| | -Tissue proliferative and wound healing properties |
| | -Anti-inflammatory, anticancer, DNA protective and antioxidant |
| **Buckwheat** | -High bactericidal properties |
| | -Protects DNA from chemical and oxidative stress (better than manuka) [29] |

Fig. 1 shows a tissue biopsy specimen and a container comprising Amber solution in a "pathologist box" under which one can view the sample under MRI. With imaging in mind, it is important the solution has a viscous nature that will stabilize the specimen from swimming in the solution - to prevent creating artefacts on MRI. This will be achieved with honey - viscosity of 10,000 cP at room temperature. Coconut oil also aids as it has a viscosity of 60 cP at room temperature. For reference, water has a viscosity of 1 cP. The solution can be kept at room temperature.

### Example 2

Four solutions (A1, A2, A3 and A4) were prepared, summarized in Table 4. Amber solutions (A1, A2 and A3) comprise LPDG, 10% honey (manufactured by Billy Bee^{™}, McCormack Canada, London Canada) and/or 1% coconut oil. All Amber derivate solutions had a yellowish color due to the honey. The pH of each solution was measured using Accument Basic Fisher Scientific, data shown in Table 4. The pH can range from about 3 to about 10.5. Lung samples were cut into small 1-2cm pieces and injected with the fixative solution before being placed in the respective fixative container. Samples were fixed at room temperature for 24 hours and at 4 degrees for 24 hours. After fixation, the samples were processed overnight in ethanol and wax. Once the samples were embedded in wax they were sent to pathology for Hematoxylin and eosin (H&E) staining. The solutions were made by heating the LPDG or sterile water and adding honey and/or coconut oil.

**Table 4. Solutions and measured pH.**

| **SOLUTIONS** | **PH** |
|---|---|
| **FORMALIN 10%** | 6.8 |
| **A1 (LPDG + 10% HONEY + 1% COCONUT OIL)** | 3.99 |
| **A2 (LPD + 10% HONEY)** | 3.94 |
| **A3 (H2O + 10% HONEY + 1% COCONUT OIL)** | 4.04 |
| **A4 (H2O + 10% HONEY)** | 4.11 |

A1, A2, and A3 Amber honey-based solutions were tested and scored higher on histology criteria than formalin.

### Example 3

A biological sample such as a lung cancer biopsy is excised during surgery or biopsy procedure. The sample is placed in a formulation described herein, optionally a honey based formulation described in Example 2. The formulation is either at about 4C or room temperature.

Within for example 24 hours, the sample is processed for imaging or immunohistochemistry.

For immunohistochemistry, the biological sample. The sample is optionally embedded in paraffin or wax or other similar agent for infusing and/or encasing the sample, optionally sliced and subjected to immunohistochemistry for a desired antigen or staining such as an H& E stain or imaged. The sample can then be processed (e.g. either the same sample or another portion of the biopsy) for nucleic acid analysis, such as RT-PCR and/or sequencing. For example, the biological sample may be subjected to DNA or RNA extraction subsequent to immunohistochemical analysis, staining, or imaging for example using a kit such as RNeasy FFPE Kit, GeneREad DNA FFPE kit or by measuring UV absorbance.

### Example 4

An Amber solution comprising 10% honey, dextran and 1% coconut oil was tested.

### Amber (comprising honey, dextran and coconut oil) preparation

To prepare Amber the honey (Terra Andes, Blueweed Hierba Azul) is first heated in a water bath at 37 degrees and then added to a low potassium dextran glucose (LPDG) solution available for example from XVIVO Perfusion AB Mässans gata 10, SE-41251 Göteborg, Sweden along with coconut oil. Amber solution is buffered to a physiological pH 7.4. The Amber solution is 10% v/v honey and 1% v/v coconut oil. Amber is kept in the fridge at 4 degrees Celsius right until use.

### Tissue collection

Seven rats of Sprague Dawley strain were collected from University Health Network. Rats are induced with 5% isoflurane in an anesthetic chamber. Once they reach a surgical plane of anesthesia, they are maintained at 5% isoflurane on a mask. 0.8mL of potassium chloride is injected IV through the tail vein under deep general anesthesia. Four organs were harvested: lung, heart, liver and kidney. Once euthanasia is confirmed, the abdomen is prepped with 70% alcohol, a laparotomy is performed to isolate and harvest both the kidney and liver. Once the abdominal organs have been collected the thoracic cavity is open and the heart and lungs are removed. All animal manipulations, including euthanasia, are completed in compliance with the guidelines set out by the Canadian Council on Animal Care.

### Tissues preparation

All tissue was cut to 2mm thin slices. Lung tissue was cut and inflated with the respective media using a 29-gauge needle to see the structural integrity on histology. The tissue was stored at 4 degrees for 24 hours. The time of storage is defined as the moment the tissue is immersed in the solution until the start of the tissue processing machine. The purpose of processing tissue after storage is to remove water from the tissue and replace it with a media like molten wax that will allow tissue to be thinly sectioned. The processing machine is composed of three steps: dehydration, clearing and infiltration. During the dehydration step, the tissue goes through a series of graded alcohol. During this step the alcohol may dissolve the lipid components of the tissue and cause shrinkage. The second step is clearing using xylene as the reagent. This transition step enables the molten wax to infiltrate the tissue since the ethanol and wax are immiscible. It should be noted that further tissue shrinkage can occur during clearing. After the tissue processing has completed, the tissue is paraffin embedded into blocks. Using a rotary microtome, the blocks were sectioned to 4 micrometers for staining.

### Histology

The tissues were stained with hematoxlin and eosin (H&E) for histological examination.

### Immunohistochemistry

One antigen was chosen as a respective marker for each organ shown in table 5. The representative marker for lung tissue is Thyroid Transcription Factor (TTF-1), a nuclear marker staining alveolar type 1 and type 2, club cells and ciliated epithelia cells. Muscle Specific Actin (MSA) was chosen for cardiac muscle, staining myocardial, myoepithelial cells and pericytes of small vessels. The marker for liver tissue is Hepatocyte Specific Antigen (HepPar-1) a mitochondrial antigen of hepatocytes. Lastly, the marker for kidney is, Common Acute Lymphoblastic Leukemia Antigen (CD10) present in glomerular epithelium. The secondary antibody for these 4 markers is Biotinylated Horse anti-Mouse.

### Immunofluorescence

Immunofluorescence staining was done on all four organs testing three common antibodies: Ve-cadherin, Vimentin (Vim) and Alpha Smooth Muscle Actin (α-SMA) shown in table 5. Ve-cadherin is a cellular adhesion molecule and was chosen as an endothelia marker. The secondary antibody used here was goat anti-rabbit IgG Alexa Fluor^{®}555 (cat # A32732). VIM is a type III intermediate filament protein expressed in mesenchymal cells. The VIM used here is primary conjugated antibody. *α* -SMA is component of the cytoskeleton structural network It is expressed in smooth muscle cells (SMCs) of blood vessels, myoepithelial and myofibroblasts cells. The secondary antibody is Donkey anti-Rabbit IgG Alexa Fluor Plus 488 (cat # A32766). The sections were counterstained with DAPI, a nuclear marker.

**Table 5: Immunohistochemistry and Immunofluorescence antibodies.**

| | **Primary Antibody** | **Manufacturer** | **Cat No.** | **Clone** | **Antigen Retrieval** | **Antibody Dilution & Incubation time** |
|---|---|---|---|---|---|---|
| IHC | | | | | | |
| | TTF-1 | Leica | NCL-L TTF1 | SPT24 | Citrate pH 6 | F: 1:300 (1 hr) A: 1:50 (1 hr) |
| | MSA | Dako | M0635 | HNF35 | Low temp Tris-EDTA pH 9 | 1:400 (overnight) |
| | HepPar-1 | Dako | M7158 | Ochiles | Tris**-** EDTA pH 9 | 1:150 (overnight) |
| | CD10 | Leica | CD10-270 | 56C6 | Low temp Tris EDTA | 1:100 (1 hr) |

| IF | | | | | | |
|---|---|---|---|---|---|---|
| | Ve-Cadherin | ThermoFisher Scientific | 36-1900 | polyclonal | Citrate pH 6 | 1:50 (overnight) |
| | VIM | Sigma | C9080 | V9 | Citrate pH 6 | 1:200 (overnight) |
| | *α* - SMA | Agilent | M0851 | 1A4 | Citrate pH 6 | 1:200 (overnight) |

### Evaluation

The quality of histology and immunohistochemistry was reviewed by a pathologist at UHN Laboratory Medicine Program. The evaluation was performed blinded and scored from 0-3 based on standardized criteria. The H&E criteria for nuclear and cytoplasmic quality is: 0: Complete loss of tissue architecture, 1: Poor cell and tissue structure but tissue type identifiable, 2: suboptimal cytology but interpretable and 3: Sharp staining and structure. Immunohistochemistry was evaluated for staining intensity: 0: No staining, 1: Patchy staining that would be clinically inadequate but present, 2: Suboptimal but interpretable staining, weak intensity and 3: Perfect staining. Statistical test was performed on GraphPad Prism 9. Non-parametric Mann Whitney U test was used for evaluation with a significance level of 5.

### Results

### Formulation of Amber

The biochemistry profile of Amber, LPDG and a 10% formalin fixative solution was determined as shown in table 6. The natural pH of Amber is 4. The preliminary experiments tested tissue storage for 24 hours at 4 degrees Celsius with Amber pH 4.. It was observed that kidney cell architecture was not well preserved and loss of nuclei within the cortex. The kidney organ is a regulator of the body's pH. With this in mind, Amber was buffered using dilute sodium hydroxide (NaOH) to a physiological pH 7.4 to optimally preserve tissue. The low potassium in LPDG is an extracellular solution to preserve epithelial and endothelial structural and functional integrity of cells. 10% Formalin is 10% buffered formalin (manufactured by Fischer chemical SF100-4 Lot 206880).

**Table 6: Biochemistry Profiles**

| Component | 10% Formalin | LPDG | Amber ph 7.4 |
|---|---|---|---|
| Sodium | 118 | 138 | 122 |
| Potassium | <1 | 6 | 8.8 |
| Chloride | <20 | 142 | 127 |
| Magnesium | <0.25 | 0.8 | 0.86 |
| Phosphate | 73.66 | 0.8 | 1.00 |
| Calcium | <0.5 | 0.3 | 0.55 |
| Bicarbonate | <5 | 1 | <5 |
| Glucose | <0.3 | 0.9 | 290 |
| Viscosity | 0.92 cP | 2.39 cP | 2.82 cP |
| pH | 7 | 7.2 | 7.4 |
| Osmolality | 1982 mOsm/kg | 297 mOsm/kg | 1585 mOsm/kg |

| | | | |
|---|---|---|---|
| *component units in mmol/L | | | |

### Histology

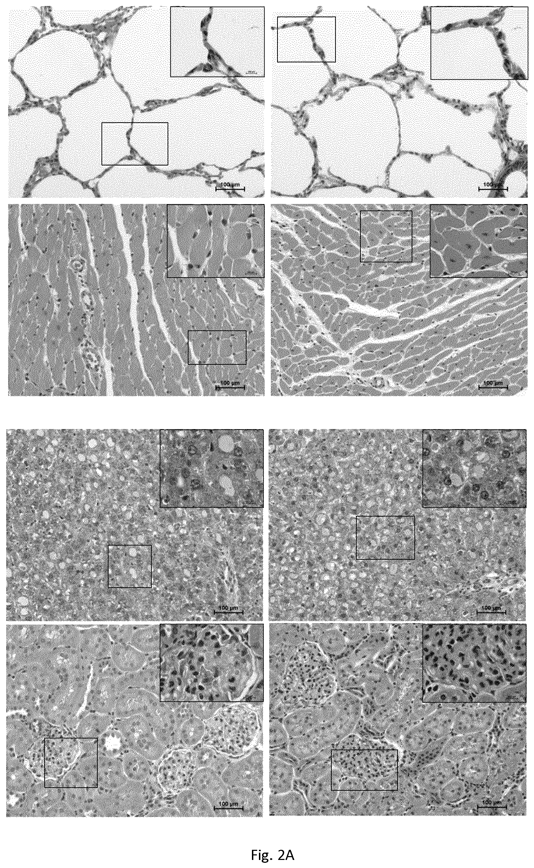

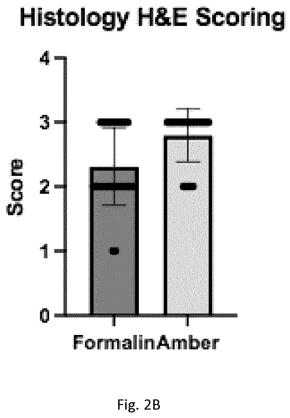

The hematoxylin and eosin photomicrographs are shown in Figure 2A. The tissue stored in Amber shows superior histology to the formalin storage, the clinical standard. The p-value for H&E staining was 0.0018 with a 5% significance level. Figure 2B shows the medians of the formalin and amber stored samples. Amber had a median of 3 and formalin 2. There were no signs of autolysis or putrefaction in Amber. The Amber group showed excellent cytoplasmic and strong nuclear staining with good preservation of tissue. Lung tissue showed sharper staining in the Amber group in comparison to Formalin storage. Lung alveoli was consistently well preserved and structurally intact in Amber storage, while the formalin group allowed for airspace collapse. Heart tissue stored in Amber showed significantly better cell distinction that formalin. However, it was observed that heart tissue stored in Amber showed slightly shrunken cells. This effect could be due to two possible reasons: difference in osmolarity between Amber and formalin or tissue shrinkage during the processing machine steps. Liver tissue showed no difference between both amber and formalin stored tissue. Kidney tissue stored in formalin had signs of cell swelling. In contrast, the amber group showed no signs of swelling, demonstrated crisp cell borders and very clear staining of glomerulus.

### Immunofluorescence

Immunofluorescence (IF) uses fluorophores in the detection of cellular proteins. IF staining is restrictive to paraffin embedded blocks, particularly formalin fixed paraffin embedded, even with antigen retrieval. Instead, fresh frozen (FF) embedded in OCT media is used, however the drawback is poor morphology of tissue and high background noise. Sectioning FF Oct embedded lung tissue on cryostat microtome is major challenge as the airways are not fixed and therefore sectioning ends up damaging the tissue section. Amber preserved tissue in paraffin blocks demonstrated excellent IF staining, providing an efficient and effective new pathway to IF staining.

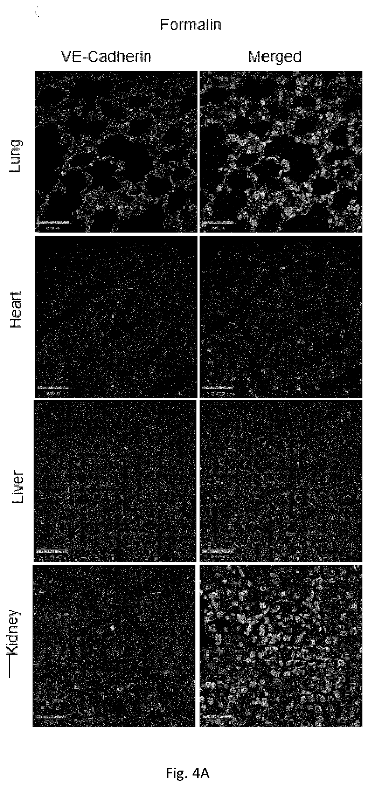

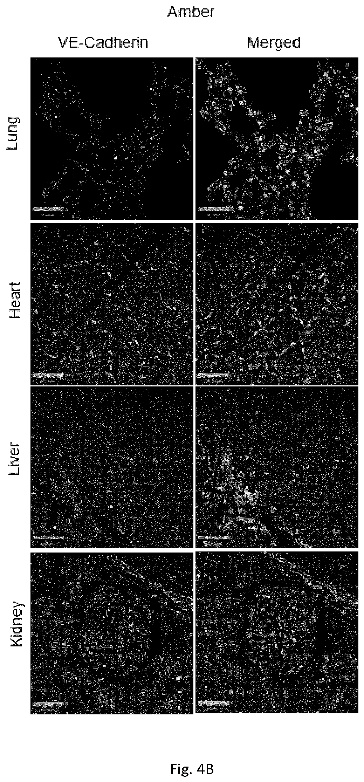

A significant finding was the increased antigenicity of endothelia marker Ve-Cadherin in the Amber groups shown in Figure 4. Cadherins are a family of type-1 transmembrane proteins of adhesion molecules which exhibit cation- dependent homophilic and heterophilic binding. Ve-cadherin is an endothelial adhesion molecule found between the junction of endothelial cells. The exposure time for Ve-cadherin staining was 500ms and 300ms for DAPI for all groups. The lung tissue stored in formalin showed high background noise and non-specific staining, while the Amber group had very sharp staining of the capillaries. Heart tissue staining was equivalent between the formalin and Amber groups; however, Amber had much stronger intensity at the same exposure time. Liver tissue stored in formalin exhibited non-specific staining, while the Amber group showed positive staining of the ECs. The kidney group stored in Amber showed prominent staining in the glomerulus capillaries, which is weaker in the formalin group.

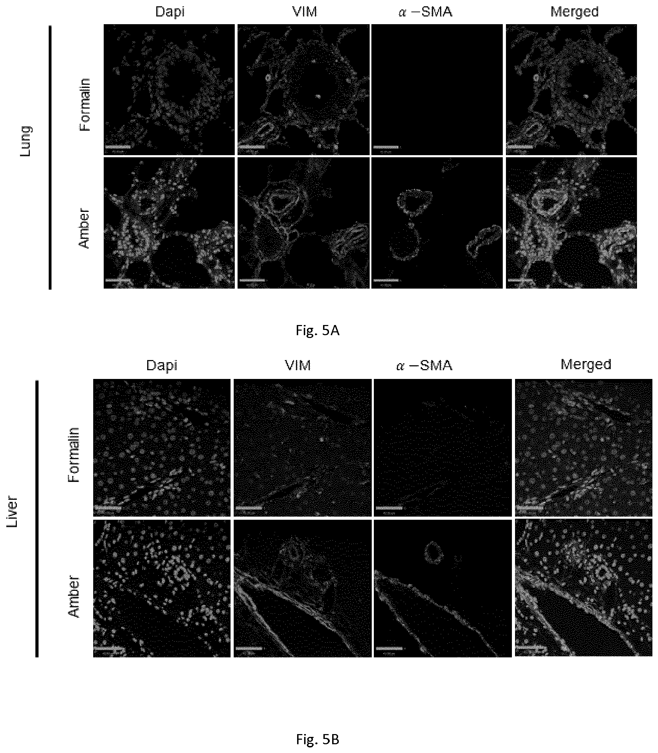

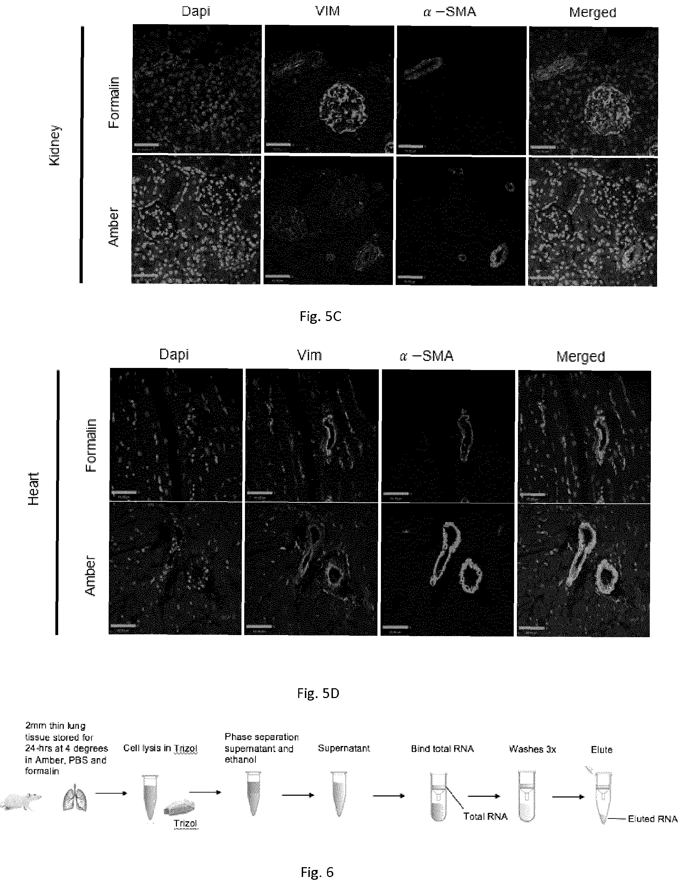

Vim and α-SMA staining is shown in Figure 5. The exposure time of VIM and α-SMA was 300ms for all groups. VIM, located intracellularly, showed high intensity staining of endothelial cells and vascular smooth muscle cells of lung vessel and capillaries of both Amber and formalin group. α-SMA was negative in the formalin lung airways and vessels. In contrast, Amber showed true positive staining. VIM showed high expression in amber stored liver tissue of the vessels in comparison to formalin. Additionally, amber liver tissue showed intense staining for α-SMA in the vessel, in comparison to formalin. Amber kidney tissue showed specific VIM staining within the glomerulus endothelial cells capillaries while the formalin group shows intense glomerulus staining. Dapi was much stronger in Amber. The α-SMA showed equivalent staining in both formalin and amber kidney tissue. Heart tissue showed equivalent VIM staining pattern of fibroblasts and endothelial cells of amber and formalin group. α-SMA expressed much more intense staining of the vessel in Amber.

### RNA Extraction

RNA is thermodynamically a stable molecule; however, it is rapidly digested in presence of the predominant RNase enzymes. RNA is most susceptible to degradation from the point of excision. In clinical setting when nucleic acid testing is needed, a tissue biopsy will be placed in a container of PBS and will be sent for same-day testing. The RNA quantity and quality of rat lung tissue was tested after 24-hour storage at 4 degrees in Amber, formalin and PBS (with no tissue processing). Qiagen RNeasy MINI kit with trizol method was used. Figure 6 illustrates the RNA extraction flowchart. RNA quantity was measured on NanoDrop spectrophotometer. The RNA quality RIN measurement was performed at MaRS Genomics centre (9^{th} floor) using the Agilent 2100 bioanalyzer. Table 7 highlights the results. It is clear that Amber stored tissue preserves greater RNA quantity, 368.46ng/ *µ*g in comparison to formalin, 13.04 ng/*µ*g and PBS, 185.46 ng/*µ*g. These results further confirm the preservation (rather than fixation) ability of Amber. The RNA quality is measured by an RNA integrity number (RIN) which is a score from 1 - 10, with 10 being complete intact RNA. PBS and Amber show equal semi-intact RNA at a score of 5.4 and 5.6 respectively. These results highlight the multi downstream use of Amber as a preservation media from the moment of tissue excision.

**Table 7: RNA quantity and quality results**

| Sample | Weight (mg) | RNA quantity ng/*µ*g | A260/280 | RNA Integrity number (RIN) |
|---|---|---|---|---|
| 10%Formalin | 12.8 | 13.04 | 1.46 | N/A* |
| Standard PBS | 12 | 185.46 | 2.02 | 5.4 |
| Amber (same as described herein) | 13.4 | 368.46 | 2.06 | 5.6 |

| | | | | |
|---|---|---|---|---|
| *N/A indicates not measurable | | | | |

While the present application has been described with reference to what are presently considered to be the preferred examples, it is to be understood that the application is not limited to the disclosed examples. To the contrary, the application is intended to cover various modifications and equivalent arrangements included within the spirit and scope of the appended claims.

The scope of the claims should not be limited by the preferred embodiments and examples, but should be given the broadest interpretation consistent with the description as a whole. The scope of the present invention is defined by the appended claims.

### CITATIONS FOR REFERENCES REFERRED TO IN THE SPECIFICATION

[1]Daugaard I, Kjeldsen T, Hager H, Hansen L, Wojdacz T. "The Influence of DNA Degradation in Formalin-Fixed, Paraffin-Embedded (FFPE) Tissue on Locus-Specific Methylation Assessment by MS-HRM". Aarhus University, Denmark. Experimental and Molecular Pathology. 2015; Volume 99, Issue 3.
[2]Elshaer N, Mahmoud M. "Toxic Effects of Formalin-treated Cadaver on Medical Students, Staff Members, and Workers in the Alexandria Faculty of Medicine". Alexandra Journal of Medicine. (2017) Vol 53, Iss 4. Pages 337-343.
[3]Kothmaier H, Rohrer D, Stacher E, Quehenberger F, Becker KF, Popper HH. "Comparison of Formalin-Free Tissue Fixatives- A Proteomic Study Testing Their Application for Routine Pathology and Research". Arch Pathol Lab Med, Medical University of Graz, Austria. 2011;135:744-752.
[4] Protection of Workers from the Risks Related to Exposure to Carcinogens or Mutagens at Work. 2018. European Parliament. http://www.europarl.europa.eu/doceo/document/TA-8-2019-0307 EN.html#title2
[5]Guyard D, Boyez A, Puhals A, et al,. "DNA Degrades During Storage in Formalin-fixed and Paraffin-Embedded Tissue". Créteil, France. Virchows Arch. 2017;471(4):491-500.
[6]Zhang P, Lehmann B, Shyr Y, Guo Y. "The Utilization of Formalin Fixed-Paraffin-Embedded Specimens in High Throughput Genomic Studies." International Journal of Genomics. 2017, vol. 2017, Article ID 19266304.
[7]McKinney M, Moon S, Kulesh D, Larsen T, Schoepp R. "Detection of Viral RNA From Paraffin-Embedded Tissues After Prolonged Formalin Fixation." Journal of Clinical Virology. 2009; Volume 44, Issue 1.
[8]Lawson MH, Rassal DM, Cummings NM, et al,. "Tissue Banking of Diagnostic Lung Cancer Biopsies for Extraction of High Quality RNA". Cancer Research UK Cambridge Research Institute. J Thorac Oncol. 2010; 5(7):956-63.
[9] Hirt S, Wahlers T, Jurmann M, Dammenhayn L, Kemnitz J, Rhode R, Haverich A. "University of Wisconsin Versus Modified Euro-Collins Solution for Lung Preservation." Ann Thorac Surg 1992;53:74-9.
[10] Delfour C, Roger P, Bret C, et al,. "RCL2, a New Fixative, Preserves Morphology and Nucleic Acid Integrity in Paraffin-Embedded Breast Carcinoma and Microdissected Breast Tumor Cells". Toulouse, France. J Mol Diagn. 2006; 8(2): 157-169.
[11] Sarot E, Carillo-Baraglioli M, Duranthon F, Pequignot A, Pyronnet S. "Assessment of Alternatives to Environmental Toxic Formalin for DNA Conservation in Biological Specimens". Environmental Sci-ence and Pollution Research International; Heidelbreg. Vol 24. Iss. 20, (July 2017): 16985-16993
[12] Uneyama C, Shibutani M, Masutomi N, Takagi H, Hirose M. "Methacarn Fixation for Genomic DNA Analysis in Microdissected, Paraffin-Embedded Tissue Specimen". J Histochem Cytochem. 20021 52:903-913. [PubMed]
[14] Rajanikanth M, Ravi A, Sreenath G, et al,. "Transit Fixatives: An Innovative Study". J Clin Diagn Res. 2015 Mar; 9(3): ZM01-ZM03
[13] Deng J, Liu R, Lu Q, et al,. "Biochemical Properties, Antibacterial and Cellular Antioxidant Activi-ties of Buckwheat Honey in Comparison to Manuka Honey". Food Chem. 2018. 252:243-249.
[15] Kurt YG, Kurt B, Ozcan O, et al,. "Preservative of Solution for Skeletal Muscle Biopsy Samples". Ankara, Turkey. Ann Indian Acad Neurol. 2015; 18(2):187-93.
[16] Mangus RS, Tector AJ, Agarwal A, Vianna R, Murdock P, Fridell Ja. "Compasion of Histidine-Tryptophan-Ketoglutarate Solution (HTK) and University of Wisconsin Solution (UW) in Adult Liver Transplantation." Liver Transpl. 2006; 12(2):226-30.
[17] Rosenfeldt F, Conyers R, Jablonski P, Langley L, et al. "Comparison of UW Solution and St.Thomas' Solution in the Rat: Importance of Potassium Concentration." Ann Thorac Surg 1996;61:576-84.
[18] Keshavjee S, Perrot M. "Lung Preservation". Semin Thorac Cardiovasc Surg 2004;16:300-308
[19] National Cancer Institute. Manuka Honey. https://www.cancer.gov/publications/dictionaries/cancer-drug/def/manuka-honey
[20] Miguel Mg, Antunes MD, Faleiro ML. "Honey as a Complementary Medicine". Integr Med Insights. 2017; 12: 1178633717702869.
[21] Martos I, Ferreres F, Tomás-Barberán FA. "Identification of Flavonoid Markers for the Botanical Origin of Eucalyptus Honey". J Agric Food Chem. 2000; 48(5); 1498-502.
[22] Muhammad A, Odunola OA, Ibrahim MA, et al,. "Potential Biological Activity of Acacia Honey". Front Biosci (Elite Ed). 2016. 1;8:351-7.

## Claims

1. A fixative composition comprising from at least 5 percent to 50 percent honey v/v of the final composition, dextran, sodium, potassium, chlorine, magnesium, sulfate, phosphate, calcium, and bicarbonate wherein the dextran is at least 10g/L to about 60g/L; the sodium is about 80mmol/L to about 190mmol/L; the potassium is about 1mmol/L to about 15mmol/L; the chlorine is about 50mmol/L to about 150mmol/L; the magnesium is about 0.1mmol/L to about 3mmol/L; the sulfate is about 0.1mmol/L to about 3mmol/L; the phosphate is about 0.1mmol/L to about 3mmol/L; the calcium is about 0.1mmol/L to about 3mmol/L; and the bicarbonate is about 0.1mmol/L to about 15mmol/L.

2. The fixative composition of claim 1, wherein the honey is selected from manuka honey, alfalfa honey, eucalyptus honey, acacia honey, or buckwheat honey or a combination of honeys described therein and/or the composition comprises about 10% honey v/v of the final composition.

3. The fixative composition of claims 1 or 2, further comprising coconut oil, wherein the composition comprises at least 0.5 to 15 percent coconut oil v/v of the final composition, optionally 1 percent coconut oil v/v of the final composition.

4. The fixative composition of any one of claims 1 to 3, wherein the concentration of one or more of the components of the composition, optionally all of the components, is
i)
sodium at 138 mmol/L
potassium at 6 mmol/L
chloride at 142 mmol/L
magnesium at 0.8 mmol/L
sulphate at 0.8 mmol/L
phosphate at 0.8 mmol/L
calcium at 0.3 mmol/L
bicarbonate at 1 mmol/L
dextran 40 at 50 g/L
glucose at 0.9 g/L
raffinose at 30 mmol/L
lactobionate at 100 mmol/L
glutathione at 3 mmol/L
honey at 10% v/v of the final composition; and
with or without coconut oil at 1% v/v of the final composition;
and/or
ii)
sodium at 122 mmol/L
potassium at 8.8 mmol/L
chloride at 127 mmol/L
magnesium at 0.86 mmol/L
phosphate at 1 mmol/L
calcium at 0.55 mmol/L
bicarbonate at less than 5 mmol/L
glucose at 290 mmol/L;
and/or wherein the composition comprises a pH of about 3 to about 10.5.

5. The fixative composition of any one of claims 1 to 4, wherein the pH is about 7 to about 7.5, preferably about 7.4, wherein the fixative composition is for preserving a biological sample comprising DNA and/or RNA, is an embalming fluid and/or is for fixing tumor DNA and/or RNA.

6. The fixative composition of claim 5, wherein the biological sample is or comprises cancer cells, and/or is a tissue sample.

7. The fixative composition of claim 5 or 6, wherein the biological sample is a biopsy, wherein the fixative composition further comprises a RNAse inhibitor or a DNAse inhibitor, is sterile and/or is echogenic.

8. The fixative composition of any one of claims 1 to 5, wherein
the dextran is about 50 g/L, and/or
the honey is about 10 percent v/v of the final composition
or the fixative composition of any one of claims 3 to 7, wherein
the dextran is about 50 g/L,
the coconut oil is about 1% v/v of the final composition; and/or
the honey is about 10 percent v/v of the final composition.

9. A method of making a fixative composition of claim 1, the method comprising combining at least 5 percent to 50 percent honey v/v of the final composition, with low potassium dextran glucose solution (LPDG), wherein LPDG comprises the dextran that is at least 10g/L to about 60g/L; the sodium that is about 80mmol/L to about 190mmol/L; the potassium that is about 1mmol/L to about 15mmol/L; the chlorine that is about 50mmol/L to about 150mmol/L; the magnesium that is about 0.1mmol/L to about 3mmol/L; the sulfate that is about 0.1mmol/L to about 3mmol/L; the phosphate that is about 0.1mmol/L to about 3mmol/L; the calcium that is about 0.1mmol/L to about 3mmol/L; and the bicarbonate that is about 0.1mmol/L to about 15mmol/L, optionally adding coconut oil, and either heating the components prior to combining or once combined.

10. The method of claim 9, wherein the fixative composition is formulated to comprise the components and concentrations as described in claim 4 or comprises LPDG and 10% honey v/v of the final composition or LPDG and 10% honey v/v of the final composition and 1% coconut oil v/v of the final composition.

11. A method of preserving a biological sample comprising immersing the biological sample in the fixative composition of any one of claims 1 to 8.

12. The method of claim 11, wherein the biological sample is fixed for at least 8 hours, at least 10 hours, at least 12 hours, at least 14 hours or at least or up to 48 hours, at about 4°C; wherein the biological sample comprises cancer cells and/or wherein the biological sample is a tissue; and optionally wherein the biological sample is assessed by an immunohistochemical analysis, in situ hybridization, immunofluorescence analysis, histological examination, and/or a nucleic acid analysis, optionally DNA extraction and/or PCR analysis.

13. A container comprising the fixative composition of any one of claims 1 to 8.

14. The container of claim 13, wherein the container further comprises a biological sample and/or is labelled; wherein the container and/or the fixative composition is sterile; wherein the container is a pathologist's box.

15. A kit comprising one or more containers of claim 13 or 14, wherein the kit further comprises an instrument for obtaining biopsies, and/or a pathologist box and instructions for use.

## Patentansprüche

1. Fixierungszusammensetzung, umfassend von mindestens 5 bis 50 Vol./Vol.-Prozent Honig der Endzusammensetzung, Dextran, Natrium, Kalium, Chlor, Magnesium, Sulfat, Phosphat, Calcium und Bicarbonat, wobei das Dextran mindestens 10 g/l bis etwa 60 g/l beträgt; das Natrium etwa 80 mmol/l bis etwa 190 mmol/l beträgt; das Kalium etwa 1 mmol/l bis etwa 15 mmol/l beträgt; das Chlor etwa 50 mmol/l bis etwa 150 mmol/l beträgt; das Magnesium etwa 0,1 mmol/l bis etwa 3 mmol/l beträgt; das Sulfat etwa 0,1 mmol/l bis etwa 3 mmol/l beträgt; das Phosphat etwa 0,1 mmol/l bis etwa 3 mmol/l beträgt; das Calcium etwa 0,1 mmol/l bis etwa 3 mmol/l beträgt; und das Bicarbonat etwa 0,1 mmol/l bis etwa 15 mmol/l beträgt.

2. Fixierungszusammensetzung nach Anspruch 1, wobei der Honig aus Manuka-Honig, Alfalfa-Honig, Eukalyptushonig, Akazienhonig oder Buchweizenhonig oder einer Kombination von darin beschriebenen Honigen ausgewählt ist und/oder die Zusammensetzung etwa 10 Vol./Vol.-% Honig der Endzusammensetzung umfasst.

3. Fixierungszusammensetzung nach Anspruch 1 oder 2, ferner umfassend Kokosnussöl, wobei die Zusammensetzung mindestens 0,5 bis 15 Vol./Vol.-% Kokosnussöl der Endzusammensetzung, optional 1 Vol./Vol.-% Kokosnussöl der Endzusammensetzung umfasst.

4. Fixierungszusammensetzung nach einem der Ansprüche 1 bis 3, wobei die Konzentration einer oder mehrerer der Komponenten der Zusammensetzung, optional aller Komponenten, wie folgt ist:
i)
Natrium bei 138 mmol/l
Kalium bei 6 mmol/l
Chlorid bei 142 mmol/l
Magnesium bei 0,8 mmol/l
Sulfat bei 0,8 mmol/l
Phosphat bei 0,8 mmol/l
Calcium bei 0,3 mmol/l
Bicarbonat bei 1 mmol/l
Dextran 40 bei 50 g/l
Glukose bei 0,9 g/l
Raffinose bei 30 mmol/l
Lactobionat bei 100 mmol/l
Glutathion bei 3 mmol/l
Honig bei 10 Vol./Vol.-% der Endzusammensetzung; und
mit oder ohne Kokosnussöl bei 1 Vol./Vol.-% der Endzusammensetzung;
und/oder
ii)
Natrium bei 122 mmol/l
Kalium bei 8,8 mmol/l
Chlorid bei 127 mmol/l
Magnesium bei 0,86 mmol/l
Phosphat bei 1 mmol/l
Calcium bei 0,55 mmol/l
Bicarbonat bei weniger als 5 mmol/l
Glukose bei 290 mmol/l;
und/oder wobei die Zusammensetzung einen pH-Wert von etwa 3 bis etwa 10,5 umfasst.

5. Fixierungszusammensetzung nach einem der Ansprüche 1 bis 4, wobei der pH-Wert etwa 7 bis etwa 7,5, vorzugsweise etwa 7,4 beträgt, wobei die Fixierungszusammensetzung zum Konservieren einer biologischen Probe dient, die DNA und/oder RNA umfasst, eine Balsamierflüssigkeit ist und/oder zum Fixieren von Tumor-DNA und/oder -RNA dient.

6. Fixierungszusammensetzung nach Anspruch 5, wobei die biologische Probe Krebszellen ist oder umfasst und/oder eine Gewebeprobe ist.

7. Fixierungszusammensetzung nach Anspruch 5 oder 6, wobei die biologische Probe eine Biopsie ist, wobei die Fixierungszusammensetzung ferner einen RNAse-Inhibitor oder einen DNAse-Inhibitor umfasst, steril ist und/oder echogen ist.

8. Fixierungszusammensetzung nach einem der Ansprüche 1 bis 5, wobei
das Dextran etwa 50 g/l beträgt, und/oder
der Honig etwa 10 Vol./Vol.-% der Endzusammensetzung beträgt
oder Fixierungszusammensetzung nach einem der Ansprüche 3 bis 7, wobei
das Dextran etwa 50 g/l beträgt,
das Kokosnussöl etwa 1 Vol./Vol.-% der Endzusammensetzung beträgt; und/oder
der Honig etwa 10 Vol./Vol.-% der Endzusammensetzung beträgt.

9. Verfahren zum Herstellen einer Fixierungszusammensetzung nach Anspruch 1, wobei das Verfahren Kombinieren von mindestens 5 bis 50 Vol./Vol.-Prozent Honig der Endzusammensetzung mit Dextranglukoselösung mit wenig Kalium (LPDG) umfasst, wobei LPDG das Dextran umfasst, das mindestens 10 g/l bis etwa 60 g/l beträgt; das Natrium, das etwa 80 mmol/l bis etwa 190 mmol/l beträgt; das Kalium, das etwa 1 mmol/l bis etwa 15 mmol/l beträgt; das Chlor, das etwa 50 mmol/l bis etwa 150 mmol/l beträgt; das Magnesium, das etwa 0,1 mmol/l bis etwa 3 mmol/l beträgt; das Sulfat, das etwa 0,1 mmol/l bis etwa 3 mmol/l beträgt; das Phosphat, das etwa 0,1 mmol/l bis etwa 3 mmol/l beträgt; das Calcium, das etwa 0,1 mmol/l bis etwa 3 mmol/l beträgt; und das Bicarbonat, das etwa 0,1 mmol/l bis etwa 15 mmol/l beträgt, optional Hinzufügen von Kokosnussöl, und entweder Erhitzen der Komponenten vor dem Kombinieren oder nach dem Kombinieren.

10. Verfahren nach Anspruch 9, wobei die Fixierungszusammensetzung so formuliert ist, dass sie die in Anspruch 4 beschriebenen Komponenten und Konzentrationen umfasst oder LPDG und 10 Vol./Vol.-% Honig der Endzusammensetzung oder LPDG und 10 Vol./Vol.-% Honig der Endzusammensetzung und 1 Vol./Vol.-% Kokosnussöl der Endzusammensetzung umfasst.

11. Verfahren zum Konservieren einer biologischen Probe, umfassend Eintauchen der biologischen Probe in die Fixierungszusammensetzung nach einem der Ansprüche 1 bis 8.

12. Verfahren nach Anspruch 11, wobei die biologische Probe für mindestens 8 Stunden, mindestens 10 Stunden, mindestens 12 Stunden, mindestens 14 Stunden oder mindestens oder bis zu 48 Stunden bei etwa 4 °C fixiert wird; wobei die biologische Probe Krebszellen umfasst und/oder wobei die biologische Probe ein Gewebe ist; und optional wobei die biologische Probe durch eine immunhistochemische Analyse, In-situ-Hybridisierung, Immunfluoreszenzanalyse, histologische Untersuchung und/oder eine Nukleinsäureanalyse, optional DNA-Extraktion und/oder PCR-Analyse, bewertet wird.

13. Behälter, umfassend die Fixierungszusammensetzung nach einem der Ansprüche 1 bis 8.

14. Behälter nach Anspruch 13, wobei der Behälter ferner eine biologische Probe umfasst und/oder markiert ist; wobei der Behälter und/oder die Fixierungszusammensetzung steril ist; wobei der Behälter eine Pathologenbox ist.

15. Kit, umfassend einen oder mehrere Behälter nach Anspruch 13 oder 14, wobei das Kit ferner ein Instrument zum Erlangen von Biopsien und/oder eine Pathologenbox und Gebrauchsanweisungen umfasst.

## Revendications

1. Composition de fixateur comprenant d'au moins 5 pour cent à 50 pour cent de miel en v/v de la composition finale, du dextrane, du sodium, du potassium, du chlore, du magnésium, du sulfate, du phosphate, du calcium et du bicarbonate, ledit dextrane représentant au moins 10 g/l à environ 60 g/l ; ledit sodium représentant environ 80 mmol/l à environ 190 mmol/l ; ledit potassium représentant environ 1 mmol/l à environ 15 mmol/l ; ledit chlore représentant environ 50 mmol/l à environ 150 mmol/l ; ledit magnésium représentant environ 0,1 mmol/l à environ 3 mmol/l ; ledit sulfate représentant environ 0,1 mmol/l à environ 3 mmol/l ; ledit phosphate représentant environ 0,1 mmol/l à environ 3 mmol/l ; ledit calcium représentant environ 0,1 mmol/l à environ 3 mmol/l ; et ledit bicarbonate représentant environ 0,1 mmol/l à environ 15 mmol/l.

2. Composition de fixateur selon la revendication 1, ledit miel étant choisi parmi le miel de manuka, le miel de luzerne, le miel d'eucalyptus, le miel d'acacia ou le miel de sarrasin ou une combinaison de miels qui sont décrits dans le présent document et/ou ladite composition comprenant environ 10 % de miel en v/v de la composition finale.

3. Composition de fixateur selon la revendication 1 ou 2, comprenant en outre de l'huile de noix de coco, ladite composition comprenant au moins 0,5 à 15 pour cent d'huile de noix de coco en v/v de la composition finale, éventuellement 1 pour cent d'huile de noix de coco en v/v de la composition finale.

4. Composition de fixateur selon l'une quelconque des revendications 1 à 3, ladite concentration d'un ou plusieurs des composants de la composition, éventuellement tous les composants, étant
i)
du sodium à 138 mmol/l
du potassium à 6 mmol/l
du chlorure à 142 mmol/l
du magnésium à 0,8 mmol/l
du sulfate à 0,8 mmol/l
du phosphate à 0,8 mmol/l
du calcium à 0,3 mmol/l
du bicarbonate à 1 mmol/l
du dextrane 40 à 50 g/l
du glucose à 0,9 g/l
du raffinose à 30 mmol/l
du lactobionate à 100 mmol/l
du glutathion à 3 mmol/l
du miel à 10 % en v/v de la composition finale ; et
avec ou sans huile de noix de coco à 1 % en v/v de la composition finale ; et/ou
ii)
du sodium à 122 mmol/l
du potassium à 8,8 mmol/l
du chlorure à 127 mmol/l
du magnésium à 0,86 mmol/l
du phosphate à 1 mmol/l
du calcium à 0,55 mmol/l
du bicarbonate à moins de 5 mmol/l
du glucose à 290 mmol/l ;
et/ou ladite composition comprenant un pH d'environ 3 à environ 10,5.

5. Composition de fixateur selon l'une quelconque des revendications 1 à 4, ledit pH étant d'environ 7 à environ 7,5, de préférence d'environ 7,4, ladite composition de fixateur étant destinée à la conservation d'un échantillon biologique comprenant de l'ADN et/ou de l'ARN, étant un fluide d'embaumement et/ou étant destinée à la fixation d'ADN et/ou d'ARN tumoral.

6. Composition de fixateur selon la revendication 5, ledit échantillon biologique étant ou comprenant des cellules cancéreuses, et/ou étant un échantillon de tissu.

7. Composition de fixateur selon la revendication 5 ou 6, ledit échantillon biologique étant une biopsie, ladite composition de fixateur comprenant en outre un inhibiteur d'ARNase ou un inhibiteur d'ADNase, étant stérile et/ou échogène.

8. Composition de fixateur selon l'une quelconque des revendications 1 à 5,
ledit dextrane représentant environ 50 g/l, et/ou
ledit miel représentant environ 10 pour cent en v/v de la composition finale,
ou ladite composition de fixateur selon l'une quelconque des revendications 3 à 7,
ledit dextrane représentant environ 50 g/l,
ladite huile de noix de coco représentant environ 1 % en v/v de la composition finale ; et/ou
ledit miel représentant environ 10 pour cent en v/v de la composition finale.

9. Procédé de fabrication d'une composition de fixateur selon la revendication 1, le procédé comprenant la combinaison d'au moins 5 pour cent à 50 pour cent de miel en v/v de la composition finale, avec une solution de glucose et de dextrane à faible teneur en potassium (LPDG), ladite LPDG comprenant le dextrane qui représente au moins 10 g/l à environ 60 g/l ; le sodium qui représente environ 80 mmol/l à environ 190 mmol/l ; le potassium qui représente environ 1 mmol/l à environ 15 mmol/l ; le chlore qui représente environ 50 mmol/l à environ 150 mmol/l ; le magnésium qui représente environ 0,1 mmol/l à environ 3 mmol/l ; le sulfate qui représente environ 0,1 mmol/l à environ 3 mmol/l ; le phosphate qui représente environ 0,1 mmol/l à environ 3 mmol/l ; le calcium qui représente environ 0,1 mmol/l à environ 3 mmol/l ; et le bicarbonate qui représente environ 0,1 mmol/l à environ 15 mmol/l, en ajoutant éventuellement de l'huile de noix de coco, et en chauffant les composants soit avant de les combiner soit une fois combinés.

10. Procédé selon la revendication 9, ladite composition de fixateur étant formulée de manière à comprendre les composants et les concentrations tels que décrits dans la revendication 4 ou comprenant de la LPDG et 10 % de miel en v/v de la composition finale ou de la LPDG et 10 % de miel en v/v de la composition finale et 1 % d'huile de noix de coco en v/v de la composition finale.

11. Procédé de conservation d'un échantillon biologique comprenant l'immersion de l'échantillon biologique dans la composition de fixateur selon l'une quelconque des revendications 1 à 8.

12. Procédé selon la revendication 11, ledit échantillon biologique étant fixé pendant au moins 8 heures, au moins 10 heures, au moins 12 heures, au moins 14 heures ou au moins ou jusqu'à 48 heures, à environ 4°C ; ledit échantillon biologique comprenant des cellules cancéreuses et/ou ledit échantillon biologique étant un tissu ; et éventuellement ledit échantillon biologique étant évalué par une analyse immunohistochimique, une hybridation in situ, une analyse par immunofluorescence, un examen histologique et/ou une analyse d'acides nucléiques, éventuellement une extraction d'ADN et/ou une analyse par PCR.

13. Récipient comprenant la composition de fixateur selon l'une quelconque des revendications 1 à 8.

14. Récipient selon la revendication 13, ledit récipient comprenant en outre un échantillon biologique et/ou étant marqué ; ledit récipient et/ou ladite composition de fixateur étant stérile ; ledit récipient étant une boîte de pathologiste.

15. Kit comprenant un ou plusieurs récipients selon la revendication 13 ou 14, ledit kit comprenant en outre un instrument permettant d'obtenir des biopsies, et/ou une boîte de pathologiste et des instructions d'utilisation.
